# EUROPEAN PATENT APPLICATION

(11) **EP 4 248 874 A1**
(43) Date of publication of application: **27.09.2023**
(21) Application number: 23162737.3
(22) Date of filing: 17.03.2023
(51) Int. Cl.: A61B 8/00

(54) **ULTRASOUND DIAGNOSTIC SYSTEM AND METHOD FOR CONTROLLING ULTRASOUND DIAGNOSTIC SYSTEM**

(30) Priority: 24.03.2022 JP 2022048482
(71) Applicant: FUJI-FILM Corporation, Minato-ku Tokyo 106-8620 (JP)
(72) Inventor: TAKAYAMA, Hidetoshi, Kanagawa (JP); TSUBOTA, Keiji, Kanagawa (JP)
(74) Representative: Hughes, Andrea Michelle

(57) **Abstract**

Provided are an ultrasound diagnostic system and a method for controlling an ultrasound diagnostic system which can take appropriate measures in a case in which an artifact has occurred, regardless of a skill level of a user.

An ultrasound diagnostic system includes: an image input unit (11, 11a, 73) that inputs an ultrasound image; a determination unit (16, 74) that determines whether or not an artifact has occurred in the ultrasound image input to the image input unit, using a trained model trained using learning data in which a plurality of ultrasound images including artifacts and occurrence causes of the artifacts are associated with each other; and a notification unit (17) that gives a notification of an occurrence location and an occurrence cause of the artifact in the ultrasound image in a case in which the determination unit determines that the artifact has occurred.

## Description

### BACKGROUND OF THE INVENTION

### 1. Field of the Invention

The present invention relates to an ultrasound diagnostic system and a method for controlling an ultrasound diagnostic system that determines an artifact.

### 2. Description of the Related Art

In the related art, there has been known an ultrasound diagnostic system that transmits ultrasonic waves into a subject from a so-called ultrasound probe and receives ultrasound echoes from the inside of the subject to acquire an ultrasound image. There is an ultrasound image including an image that is a so-called artifact. It is known that an artifact occurs due to various factors. For example, in some cases, the artifact occurs due to the reflection of an ultrasound beam, which is a so-called side lobe and is radially transmitted from a transducer array composed of a plurality of ultrasound transducers, from a reflector in the subject.

In some cases, the artifact interferes with the observation of an anatomical structure of the subject included in the ultrasound image. In this case, it is desired to reduce the artifacts in the ultrasound image. However, since the artifacts occur due to various factors depending on the types of the artifacts, there are various countermeasures for reducing the artifacts. Therefore, as disclosed in, for example, JP2021-506470A and JP2001-501656A, a technique has been invented which specifies an artifact in an ultrasound image as any one of a plurality of predetermined types. JP2021-506470A discloses a technique which specifies an artifact in an ultrasound image as any one of a plurality of types. For example, the technique specifies whether the artifact indicates a shadow of an anatomical structure in a subject or gas accumulated in the subject. JP2001-501656A discloses a technique which specifies an artifact in an ultrasound image as any one of a plurality of types. For example, the technique specifies whether the artifact is caused by so-called multiple reflection and whether the artifact indicates a shadow of an anatomical structure.

### SUMMARY OF THE INVENTION

The artifact may interfere with the observation of the anatomical structure of the subject included in the ultrasound image. On the other hand, the artifact may be useful information for diagnosing, for example, a medical condition of the subject. In addition, in general, a user, such as a doctor, needs to have a thorough knowledge of the occurrence causes of the artifacts, in order to reduce the artifacts with, for example, an appropriate operation of changing the orientation and inclination of the ultrasound probe and to utilize information related to the type of artifacts for the diagnosis of the subject. In JP2021-506470A and JP2001-501656A, the artifact in the ultrasound image is specified as any one of a plurality of predetermined types. However, even in a case in which the user checks the specified type of artifact, for example, it may be difficult for the user to understand the detailed cause of the artifact. In particular, it may be difficult for an unskilled user to take appropriate measures for reducing the artifacts and to effectively utilize the artifacts for diagnosis.

The invention has been made to solve the problems of the related art, and an object of the invention is to provide an ultrasound diagnostic system and a method for controlling an ultrasound diagnostic system that can take appropriate measures in a case in which an artifact has occurred, regardless of a skill level of a user.

In order to achieve the above object, according to an aspect of the invention, there is provided an ultrasound diagnostic system comprising: an image input unit that inputs an ultrasound image; a determination unit that determines whether or not an artifact has occurred in the ultrasound image input to the image input unit, using a trained model trained using learning data in which a plurality of ultrasound images including artifacts and occurrence causes of the artifacts are associated with each other; and a notification unit that gives a notification of an occurrence location and an occurrence cause of the artifact in the ultrasound image in a case in which the determination unit determines that the artifact has occurred.

The ultrasound diagnostic system may further comprise a data storage unit that stores an occurrence principle of the artifact and an artifact elimination method based on the occurrence principle. In a case in which the notification unit gives the notification of the occurrence cause, the notification unit may also give a notification of the artifact elimination method based on the occurrence principle which is stored in the data storage unit.

The data storage unit may store a case in which an artifact has been effectively utilized for diagnosis. In a case in which the notification unit gives the notification of the occurrence cause, the notification unit may also give a notification of the case in which the artifact has been effectively utilized for diagnosis which is stored in the data storage unit.

The ultrasound diagnostic system may further comprise a diagnostic apparatus that includes the image input unit, the determination unit, and the notification unit. The data storage unit may be disposed in the diagnostic apparatus.

In addition, the ultrasound diagnostic system may further comprise: a diagnostic apparatus that includes the image input unit, the determination unit, and the notification unit; and a server that is connected to the diagnostic apparatus through a network. The data storage unit may be disposed in the server.

Further, the ultrasound diagnostic system may further comprise: a diagnostic apparatus that includes the notification unit; and a server that is connected to the diagnostic apparatus through a network and includes the image input unit and the determination unit. The data storage unit may be disposed in the server.

The ultrasound diagnostic system may further comprise an input device that is used to designate a region of interest in response to an input operation of a user on the ultrasound image input to the image input unit. The determination unit may determine whether or not an artifact has occurred in the region of interest.

The image input unit may include an ultrasound probe having a transducer array and an image acquisition unit that transmits and receives an ultrasound beam to and from a subject with the transducer array and acquires an ultrasound image on the basis of a received signal output from the transducer array.

The ultrasound diagnostic system may further comprise: a probe detection unit that detects an orientation and an inclination of the ultrasound probe; and a guide unit that guides changing the orientation and the inclination of the ultrasound probe such that the artifacts are reduced on the basis of the orientation and the inclination of the ultrasound probe detected by the probe detection unit.

The probe detection unit may consist of a probe sensor that is disposed in the ultrasound probe.

Further, the probe detection unit may include an optical camera that images the ultrasound probe to acquire an optical image and an optical image analysis unit that analyzes the optical image acquired by the optical camera to detect the orientation and the inclination of the ultrasound probe.

According to another aspect of the invention, there is provided a method for controlling an ultrasound diagnostic system. The method comprises: inputting an ultrasound image; determining whether or not an artifact has occurred in the input ultrasound image, using a trained model trained using learning data in which a plurality of ultrasound images including artifacts and occurrence causes of the artifacts are associated with each other; and giving a notification of an occurrence location and an occurrence cause of the artifact in the ultrasound image in a case in which it is determined that the artifact has occurred.

According to the invention, an ultrasound diagnostic system comprises an image input unit that inputs an ultrasound image, a determination unit that determines whether or not an artifact has occurred in the ultrasound image input to the image input unit, using a trained model trained using learning data in which a plurality of ultrasound images including artifacts and occurrence causes of the artifacts are associated with each other, and a notification unit that gives a notification of an occurrence location and an occurrence cause of the artifact in the ultrasound image in a case in which the determination unit determines that the artifact has occurred. Therefore, it is possible to take appropriate measures in a case in which an artifact occurs, regardless of the skill level of the user.

### BRIEF DESCRIPTION OF THE DRAWINGS

Fig. 1 is a block diagram illustrating a configuration of a diagnostic apparatus according to Embodiment 1 of the invention.
Fig. 2 is a flowchart illustrating an operation of the diagnostic apparatus according to Embodiment 1 of the invention.
Fig. 3 is a block diagram illustrating a configuration of an ultrasound diagnostic system according to Embodiment 2 of the invention.
Fig. 4 is a block diagram illustrating a configuration of a transmitting and receiving circuit in Embodiment 2 of the invention.
Fig. 5 is a block diagram illustrating a configuration of an image generation unit according to Embodiment 2 of the invention.
Fig. 6 is a block diagram illustrating a configuration of an ultrasound diagnostic system according to Embodiment 3 of the invention.
Fig. 7 is a block diagram illustrating a configuration of an ultrasound diagnostic system according to Embodiment 4 of the invention.
Fig. 8 is a block diagram illustrating a configuration of an ultrasound diagnostic system according to Embodiment 5 of the invention.
Fig. 9 is a block diagram illustrating a configuration of an ultrasound diagnostic system according to Embodiment 6 of the invention.

### DESCRIPTION OF THE PREFERRED EMBODIMENTS

Hereinafter, embodiments of the invention will be described with reference to the accompanying drawings.

The following description of components is based on a representative embodiment of the invention. However, the invention is not limited to the embodiment.

In addition, in the specification, a numerical range represented by "to" means a range including numerical values described before and after "to" as a lower limit value and an upper limit value.

In the specification, the terms "same" and "similar" include an error range generally allowed in the technical field.

### Embodiment 1

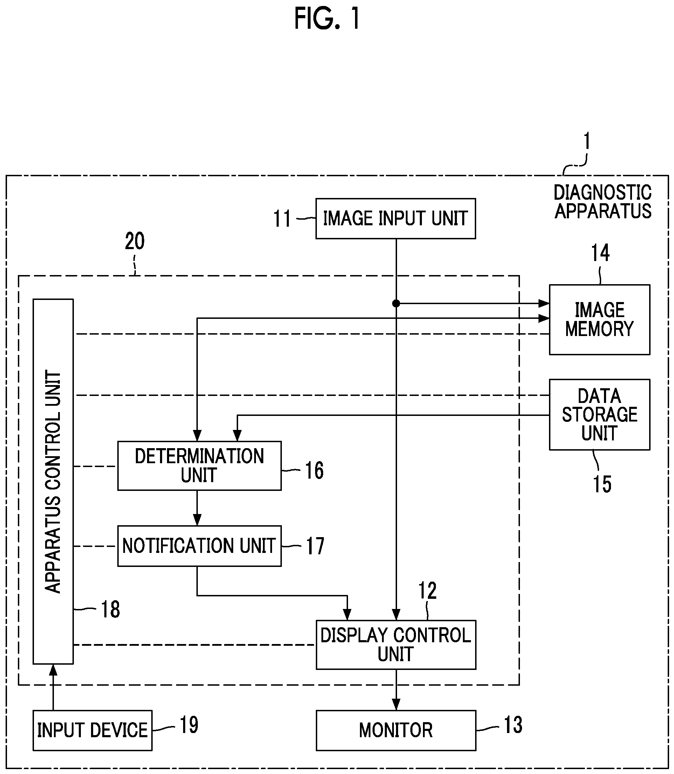

Fig. 1 illustrates a configuration of an ultrasound diagnostic system according to Embodiment 1 of the invention. The ultrasound diagnostic system according to Embodiment 1 is configured by a diagnostic apparatus 1. The diagnostic apparatus 1 displays an ultrasound image obtained from, for example, a so-called ultrasound probe, an external apparatus (not illustrated), such as a so-called ultrasound diagnostic apparatus, and an external memory (not illustrated). For example, the diagnostic apparatus 1 is used by a user, such as an examiner, to check an ultrasound image captured in real time by the ultrasound probe or is used by a user, such as a doctor, to observe the ultrasound image and to diagnose a subject.

The diagnostic apparatus 1 comprises an image input unit 11, and a display control unit 12 and a monitor 13 are sequentially connected to the image input unit 11. Further, an image memory 14 is connected to the image input unit 11. The image memory 14 is connected to the display control unit 12. In addition, the diagnostic apparatus 1 comprises a data storage unit 15. A determination unit 16 is connected to the image memory 14 and the data storage unit 15. Further, a notification unit 17 is connected to the determination unit 16. The notification unit 17 is connected to the display control unit 12.

In addition, an apparatus control unit 18 is connected to the display control unit 12, the image memory 14, the data storage unit 15, the determination unit 16, and the notification unit 17. Furthermore, an input device 19 is connected to the apparatus control unit 18. Further, the display control unit 12, the determination unit 16, the notification unit 17, and the apparatus control unit 18 constitute a processor 20 for the diagnostic apparatus 1.

The image input unit 11 inputs an ultrasound image to the diagnostic apparatus 1 from, for example, an external ultrasound probe (not illustrated), an external apparatus (not illustrated), such as an ultrasound diagnostic apparatus, or an external memory (not illustrated). The image input unit 11 includes, for example, a connection terminal for wired connection to the ultrasound probe, the external apparatus, such as the ultrasound diagnostic apparatus, or an external recording medium through a communication cable (not illustrated) and the like or an antenna for wireless connection to the ultrasound probe, the external apparatus, or the external recording medium.

A recording media, such as a flash memory, a hard disk drive (HDD), a solid state drive (SSD), a flexible disk (FD), a magneto-optical disk (MO), a magnetic tape (MT), a random access memory (RAM), a compact disc (CD), a digital versatile disc (DVD), a secure digital card (SD card), or a universal serial bus memory (USB memory), is given as an example of the external memory connected to the image input unit 11.

Under the control of the apparatus control unit 18, the display control unit 12 performs a predetermined process on, for example, the ultrasound image input by the image input unit 11 and displays the ultrasound image on the monitor 13.

The monitor 13 displays various kinds of information under the control of the display control unit 12. For example, the monitor 13 can include a display device such as a liquid crystal display (LCD) or an organic electroluminescence (EL) display.

The image memory 14 is a memory that stores the ultrasound image input by the image input unit 11 under the control of the apparatus control unit 18. The ultrasound image stored in the image memory 14 is read and transmitted to the determination unit 16 under the control of the apparatus control unit 18. In addition, the ultrasound image stored in the image memory 14 may be read, transmitted to the display control unit 12, and displayed on the monitor 13 under the control of the apparatus control unit 18. For example, a recording medium, such as a flash memory, an HDD, an SSD, an FD, a MO disk, an MT, a RAM, a CD, a DVD, an SD card, or a USB memory, is used as the image memory 14.

However, in some cases, an image that is a so-called artifact is included in an ultrasound image. It is known that the artifact occurs due to various factors. For example, in some cases, the artifact occurs due to the reflection of an ultrasound beam, which is a so-called side lobe and is radially transmitted from a transducer array composed of a plurality of ultrasound transducers, from a reflector in the subject.

In addition, a scanning method, such as so-called linear scanning or sector scanning, is generally known as an ultrasonic wave scanning method. It is known that the artifact occurring due to the side lobe has a different shape depending on, for example, the scanning method. For example, in a case in which the linear scanning is performed, the artifact occurring due to the side lobe often has a shape that is curved toward a deep portion at both ends of the ultrasound image in a lateral direction. For example, in a case in which the sector scanning is performed, the artifact occurring due to the side lobe often has a shape that is curved toward a shallow portion at both ends of the ultrasound image in the lateral direction. Here, the lateral direction of the ultrasound image means a direction orthogonal to a depth direction in the ultrasound image.

In some cases, the artifact interferes with the observation of an anatomical structure of the subject included in the ultrasound image. In this case, it is necessary to take appropriate measures according to the occurrence location and occurrence cause of the artifact in order to reduce the artifacts in the ultrasound image. For example, in a case in which the artifact occurs due to the reflection of the side lobe from the reflector in the subject, the artifacts can be reduced by changing at least one of the orientation or inclination of the ultrasound probe which is in contact with the subject such that the side lobe is not reflected from the reflector that causes the artifact.

Here, changing the orientation of the ultrasound probe means rotating the ultrasound probe such that a tomographic plane of the subject to be scanned is rotated while keeping the position of a so-called acoustic lens of the ultrasound probe on a body surface of the subject constant. Further, changing the inclination of the ultrasound probe means inclining the ultrasound probe such that the tomographic plane of the subject to be scanned is inclined, using the position of the acoustic lens of the ultrasound probe on the body surface of the subject as a fulcrum.

In addition, the artifact may be useful information for diagnosing, for example, a medical condition of the subject. For example, in a case in which an artifact, which is a so-called A-line caused by multiple reflection between the pleura and the ultrasound probe, is clearly included in an ultrasound image of the lungs of the subject, so-called pneumothorax is suspected as the medical condition of the subject. In addition, in a case in which a linear artifact which extends substantially in the depth direction is included in the ultrasound image of the lungs of the subject, so-called pneumonia or pulmonary edema is suspected as the medical condition of the subject. Further, for example, in a case in which a linear artifact which extends substantially in the depth direction is included in an ultrasound image of the liver of the subject, so-called liver cirrhosis, fatty liver, or the like is suspected as the medical condition of the subject. As described above, the user, such as the doctor, can check the artifact included in the ultrasound image to accurately diagnose the medical condition of the subject. For example, a linear artifact may occur due to a skin crease of the subject. Therefore, in general, the user needs to have a thorough knowledge of the occurrence location and occurrence cause of the artifact in order to make an accurate diagnosis.

The data storage unit 15 is a memory that stores in advance an occurrence principle of each of a plurality of different types of artifacts, an artifact elimination method based on the occurrence principle, and a case in which the artifact has been effectively utilized for diagnosis. The occurrence principle of the artifact and the artifact elimination method stored in the data storage unit 15 are read and transmitted to the determination unit 16 under the control of the apparatus control unit 18. Here, for example, a recording medium, such as a flash memory, an HDD, an SSD, an FD, an MO disk, an MT, a RAM, a CD, a DVD, an SD card, or a USB memory, is used as the data storage unit 15.

The determination unit 16 determines whether or not the artifact has occurred in the ultrasound image input to the image input unit 11, using a trained model which has been trained with learning data in which a plurality of ultrasound images including artifacts and the occurrence cause of each of the artifacts are associated with each other.

The determination unit 16 can use, as the trained model, a model that follows, for example, a so-called residual neural network (ResNet) algorithm, a so-called dense convolutional network (DenseNet) algorithm, a so-called AlexNet algorithm, a so-called baseline algorithm, a so-called batch normalization algorithm, a so-called dropout regularization algorithm, a so-called NetWidth search algorithm, or a so-called NetDepth search algorithm. In addition, the determination unit 16 can appropriately combine models that follow these algorithms and use the combinations.

Further, the determination unit 16 uses the trained model to estimate the occurrence location and occurrence cause of the artifact in the ultrasound image. The determination unit 16 can estimate an artifact having a shape, in which both ends of the ultrasound image in the lateral direction are curved toward a deep portion, as the artifact occurring due to the reflection of the side lobe from the reflector in a case in which the linear scanning is performed and can estimate the occurrence location of the artifact, that is, the position of the reflect from which the side lobe is reflected.

The determination unit 16 transmits the estimated occurrence location and occurrence cause of the artifact to the notification unit 17. In addition, the determination unit 16 reads the occurrence principle of the artifact and the artifact elimination method, which correspond to the estimated occurrence cause of the artifact, from the data storage unit 15 and transmits them to the notification unit 17.

In a case in which the determination unit 16 determines that an artifact has occurred, the notification unit 17 notifies the user of the occurrence location and occurrence cause of the artifact estimated by the determination unit 16. For example, the notification unit 17 can display a message indicating the occurrence location and occurrence cause of the artifact on the monitor 13 to notify the user of the occurrence location and occurrence cause of the artifact. In addition, the notification unit 17 can display the message on the monitor 13 using a text, a still image, a moving image, and combinations thereof.

Further, in a case in which the notification unit 17 notifies the user of the occurrence cause of the artifact, the notification unit 17 can also notify the user of the artifact elimination method based on the occurrence principle of the artifact stored in the data storage unit 15. In addition, in a case in which the notification unit 17 notifies the user of the occurrence cause of the artifact, the notification unit 17 can also notify the user of the case in which the artifact has been effectively utilized for diagnosis which is stored in the data storage unit 15.

The input device 19 receives an input operation of the user and transmits input information to the apparatus control unit 18. For example, the input device 19 is composed of devices, such as a keyboard, a mouse, a track ball, a touch pad, and a touch panel, used by the examiner to perform an input operation.

The apparatus control unit 18 controls each unit of the diagnostic apparatus according to, for example, a program recorded in advance.

In addition, the processor 20 including the display control unit 12, the determination unit 16, the notification unit 17, and the apparatus control unit 18 of the diagnostic apparatus 1 is composed of a central processing unit (CPU) and a control program for causing the CPU to perform various processes. However, the processor may be composed of a field programmable gate array (FPGA), a digital signal processor (DSP), an application specific integrated circuit (ASIC), a graphics processing unit (GPU), and other integrated circuits (ICs) or may be composed of combinations thereof.

In addition, the display control unit 12, the determination unit 16, the notification unit 17, and the apparatus control unit 18 of the processor 20 may be partially or wholly integrated into, for example, one CPU.

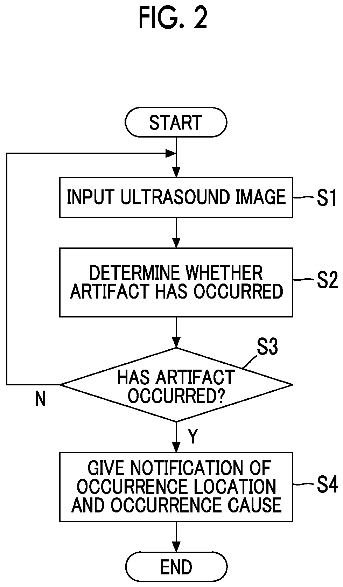

Next, an operation of the diagnostic apparatus 1 according to Embodiment 1 will be described with reference to a flowchart illustrated in Fig. 2.

First, in Step S1, an ultrasound image is input to the image input unit 11 from, for example, an external ultrasound probe (not illustrated), an external apparatus (not illustrated), or an external memory (not illustrated). The input ultrasound image is displayed on the monitor 13 through the display control unit 12. Further, the input ultrasound image is stored in the image memory 14.

Then, in Step S2, the determination unit 16 performs a process of reading the ultrasound image stored in the image memory 14 on the basis of the input operation of the user through the input device 19 or automatically and determining whether or not an artifact has occurred in the ultrasound image. In this case, the determination unit 16 performs the process of determining whether or not an artifact has occurred, using the trained model which has been trained with the learning data in which a plurality of ultrasound images including artifacts and the occurrence cause of each of the artifacts are associated with each other.

In Step S3, the determination unit 16 outputs a result of determining whether or not an artifact has occurred in the ultrasound image. Here, in a case in which the determination result indicating that the artifact has not occurred is output, the process returns to Step S1. In a case in which an ultrasound image is input to the image input unit 11, the processes in Steps S1 to S3 are performed again.

In a case in which the determination result indicating that the artifact has occurred is output in Step S3, the process proceeds to Step S4. In this case, the determination unit 16 estimates the occurrence location and occurrence cause of the artifact included in the ultrasound image, using the trained model.

In general, the user needs to have a thorough knowledge of the artifact in order to check the artifact and to estimate the occurrence location and occurrence cause of the artifact. However, according to the determination unit 16, the occurrence location and occurrence cause of the artifact are easily and automatically estimated, regardless of the skill level of the user.

In Step S4, for example, the notification unit 17 displays a message on the monitor 13 to notify the user of the occurrence location and occurrence cause of the artifact determined in Step S3. Therefore, the user can easily and accurately understand the occurrence location and occurrence cause of the artifact, regardless of the skill level thereof.

In a case in which the process in Step S4 is completed in this way, the operation of the diagnostic apparatus 1 according to the flowchart illustrated in Fig. 2 ends.

As described above, according to the diagnostic apparatus 1 of Embodiment 1, the determination unit 16 determines whether or not an artifact has occurred in the ultrasound image input to the image input unit 11, using the trained model, and estimates the occurrence location and occurrence cause of the artifact. The notification unit 17 notifies the user of the occurrence location and occurrence cause of the artifact. Therefore, the user can easily and accurately understand the occurrence location and occurrence cause of the artifact, regardless of the skill level of the user, and can take appropriate measures, such as reducing the artifacts or diagnosing the medical condition of the subject, on the basis of the artifact.

In a case in which the determination result indicating that the artifact has occurred is output in Step S3, the determination unit 16 can read an artifact elimination method corresponding to the occurrence cause of the artifact from the data storage unit 15 and transmit the artifact elimination method to the notification unit 17. In this case, in Step S4, the notification unit 17 can notify the user of the occurrence cause of the artifact and the method for eliminating the artifact. For example, in a case in which an external ultrasound probe (not illustrated) is connected to the image input unit 11, the user can change the orientation or inclination of the ultrasound probe with reference to the artifact elimination method notified by the notification unit 17 to eliminate the artifact.

In addition, in a case in which the determination result indicating that the artifact has occurred is output in Step S3, the determination unit 16 can read the case in which the artifact has been effectively utilized for diagnosis, which corresponds to the occurrence cause of the artifact, from the data storage unit 15 and transmit the case to the notification unit 17. In this case, in Step S4, the notification unit 17 can notify the user of the case in which the artifact has been effectively utilized for diagnosis together with the occurrence cause of the artifact. The user, such as the doctor, can accurately diagnose, for example, the medical condition of the subject with reference to the case notified by the notification unit 17.

Further, the configuration in which the notification unit 17 displays a message on the monitor 13 to notify the user of the occurrence location and occurrence cause of the artifact has been described. However, the method for notifying the user performed by the notification unit 17 is not particularly limited. For example, in a case in which the diagnostic apparatus 1 includes a speaker (not illustrated), the notification unit 17 can output a voice through the speaker to notify the user.

Furthermore, the configuration in which the determination unit 16 determines whether or not an artifact has occurred in the entire ultrasound image has been described. However, for example, the determination unit 16 may determine whether or not an artifact has occurred in a region of interest designated in the ultrasound image in response to the input operation of the user through the input device 19. The determination unit 16 determines whether or not an artifact has occurred in the region of interest, which makes it possible to reduce a calculation load and to more quickly determine whether or not an artifact has occurred, as compared to a case in which it is determined whether or not an artifact has occurred in the entire ultrasound image.

### Embodiment 2

The ultrasound diagnostic system according to the invention may also comprise the ultrasound probe connected to the diagnostic apparatus 1.

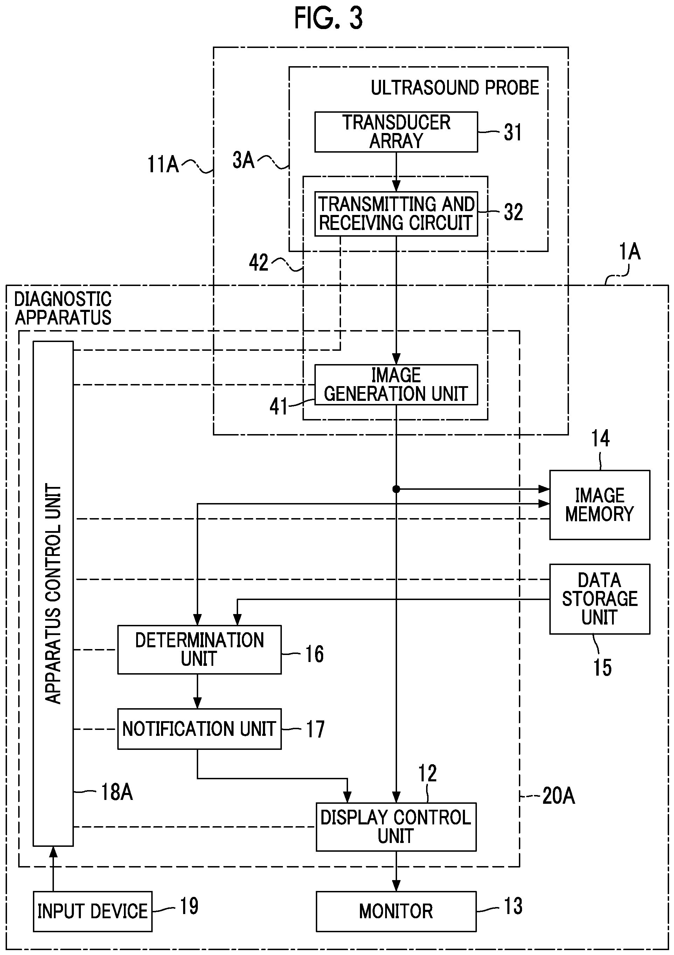

Fig. 3 illustrates a configuration of an ultrasound diagnostic system according to Embodiment 2 of the invention. The ultrasound diagnostic system according to Embodiment 2 differs from the ultrasound diagnostic system including the diagnostic apparatus 1 illustrated in Fig. 1 in that it comprises a diagnostic apparatus 1A instead of the diagnostic apparatus 1 and an ultrasound probe 3A connected to the diagnostic apparatus 1A is added.

The ultrasound probe 3A comprises a transducer array 31 and a transmitting and receiving circuit 32 that is connected to the transducer array 31.

The diagnostic apparatus 1A differs from the diagnostic apparatus 1 according to Embodiment 1 in that it comprises an image generation unit 41 instead of the image input unit 11 and comprises an apparatus control unit 18A instead of the apparatus control unit 18. In the diagnostic apparatus 1A, the image generation unit 41 is connected to the transmitting and receiving circuit 32 of the ultrasound probe 3A. Here, the ultrasound probe 3A and the image generation unit 41 constitute an image input unit 11A. In addition, the transmitting and receiving circuit 32 and the image generation unit 41 constitute an image acquisition unit 42. Further, the image generation unit 41 is connected to the display control unit 12, the image memory 14, and the apparatus control unit 18A. Furthermore, the apparatus control unit 18A is connected to the transmitting and receiving circuit 32 of the ultrasound probe 3A. Moreover, the image generation unit 41, the display control unit 12, the determination unit 16, the notification unit 17, and the apparatus control unit 18A constitute a processor 20A for the diagnostic apparatus 1A.

The transducer array 31 of the ultrasound probe 3A has a plurality of ultrasound transducers which are arranged one-dimensionally or two-dimensionally. Each of the ultrasound transducers transmits ultrasonic waves in response to a driving signal supplied from the transmitting and receiving circuit 32. In addition, each of the ultrasound transducers receives ultrasound echoes from the subject and outputs a signal based on the ultrasound echoes. For example, each of the ultrasound transducers is configured by forming electrodes at both ends of a piezoelectric body consisting of piezoelectric ceramic typified by lead zirconate titanate (PZT), a polymer piezoelectric element typified by polyvinylidene difluoride (PVDF), or a piezoelectric single crystal typified by lead magnesium niobate-lead titanate (PMN-PT).

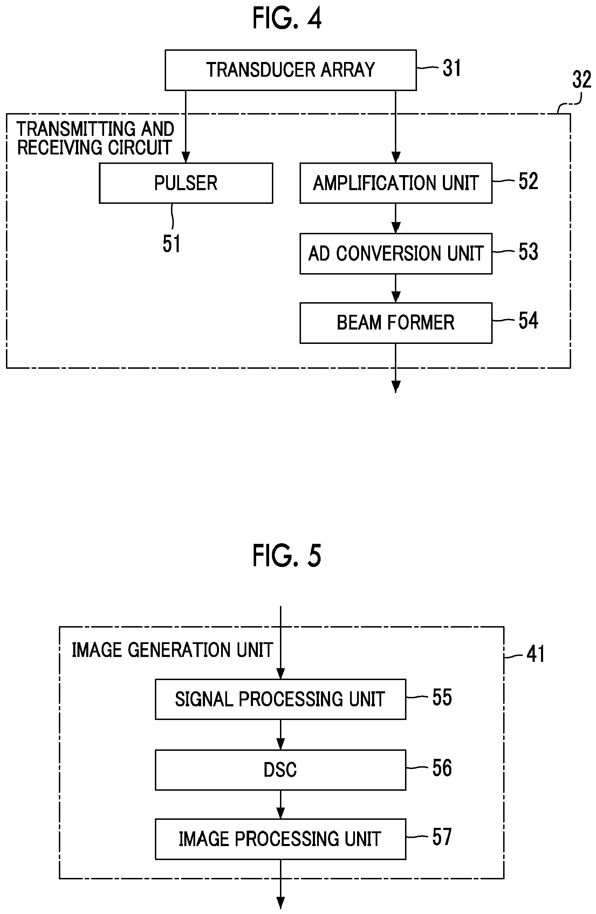

The transmitting and receiving circuit 32 transmits ultrasonic waves from the transducer array 31 and generates a sound ray signal on the basis of a received signal acquired by the transducer array 31 under the control of the apparatus control unit 18A. As illustrated in Fig. 4, the transmitting and receiving circuit 32 includes a pulser 51 that is connected to the transducer array 31, and an amplification unit 52, an analog-to-digital (AD) conversion unit 53, and a beam former 54 that are sequentially connected in series to the transducer array 31.

The pulser 51 includes, for example, a plurality of pulse generators and supplies each driving signal to the plurality of ultrasound transducers while adjusting the amount of delay such that the ultrasonic waves transmitted from the plurality of ultrasound transducers of the transducer array 31 form an ultrasound beam, on the basis of a transmission delay pattern selected in response to a control signal from the apparatus control unit 18A. As described above, in a case in which a pulsed or continuous-wave voltage is applied to the electrodes of the ultrasound transducers of the transducer array 31, the piezoelectric body is expanded and contracted, and pulsed or continuous ultrasonic waves are generated from each ultrasound transducer. An ultrasound beam is formed from a combined wave of the ultrasonic waves.

The transmitted ultrasound beam is reflected from a target, such as a part of the subject, and is propagated toward the transducer array 31 of the ultrasound probe 3A. The ultrasound echoes propagated toward the transducer array 31 in this way are received by each of the ultrasound transducers constituting the transducer array 31. In this case, each of the ultrasound transducers constituting the transducer array 31 receives propagated ultrasound echoes, is expanded and contracted to generate a received signal which is an electric signal, and outputs the received signal to the amplification unit 52.

The amplification unit 52 amplifies the signal input from each of the ultrasound transducers constituting the transducer array 31 and transmits the amplified signal to the AD conversion unit 53. The AD conversion unit 53 converts the signal transmitted from the amplification unit 52 into digital received data. The beam former 54 applies a delay to each received data item received from the AD conversion unit 53 to perform a so-called reception focus process. Each received data item converted by the AD conversion unit 53 is phase-adjusted and added, and a sound ray signal in which the focus of the ultrasound echo has been narrowed down is acquired by this reception focus process.

As illustrated in Fig. 5, the image generation unit 41 has a configuration in which a signal processing unit 55, a digital scan converter (DSC) 56, and an image processing unit 57 are sequentially connected in series to each other.

The signal processing unit 55 corrects the attenuation of the sound ray signal received from the transmitting and receiving circuit 32 caused by a distance according to the depth of the reflection position where the ultrasonic waves are reflected, using a sound velocity value set by the apparatus control unit 18A, and then performs an envelope detection process on the sound ray signal to generate a B-mode image signal which is tomographic image information related to the tissues in the subject.

The DSC 56 converts the B-mode image signal generated by the signal processing unit 55 into an image signal following a normal television signal scanning method (raster conversion).

The image processing unit 57 performs various types of necessary image processing including a gradation process on the B-mode image signal input from the DSC 56 and then transmits the B-mode image signal to the display control unit 12 and the image memory 14. Hereinafter, the B-mode image signal subjected to the image processing by the image processing unit 57 is simply referred to as an ultrasound image.

A plurality of frames of ultrasound images continuously generated by the image generation unit 41 are sequentially displayed on the monitor 13 and stored in the image memory 14. The determination unit 16 reads the ultrasound image from the image memory 14 and determines whether or not an artifact has occurred using the trained model. In a case in which it is determined that an artifact has occurred, the determination unit 16 estimates the occurrence location and occurrence cause of the artifact using the trained model. For example, the notification unit 17 displays a message on the monitor 13 to notify the user of the occurrence location and occurrence cause of the artifact estimated by the determination unit 16.

As described above, according to the ultrasound diagnostic system of Embodiment 2 of the invention, for example, the user can check the occurrence location and occurrence cause of the artifact notified by the notification unit 17 to easily change the orientation, inclination, and the like of the ultrasound probe 3A such that the artifact in the ultrasound image is eliminated. In addition, for example, the user can check the occurrence location and occurrence cause of the artifact notified by the notification unit 17 to diagnose the subject while easily and effectively utilizing the information of the artifact.

Further, the notification unit 17 may notify the user of the occurrence principle of the artifact and the artifact elimination method based on the occurrence principle, or the case in which the artifact has been effectively utilized for diagnosis which is stored in advance in the data storage unit 15. Therefore, the user can more easily change, for example, the orientation and inclination of the ultrasound probe 3Ain order to eliminate the artifact and can diagnose the subject while more easily and effectively utilizing the information of the artifact.

In the above-described embodiment, the image generation unit 41 is comprised in the diagnostic apparatus 1A. However, the image generation unit 41 may be comprised in the ultrasound probe 3A instead of being comprised in the diagnostic apparatus 1A. In this case, for example, the diagnostic apparatus 1A comprises the image input unit 11 as in the diagnostic apparatus 1 according to Embodiment 1 illustrated in Fig. 1, and an ultrasound image generated by the ultrasound probe 3A can be input to the image input unit 11.

Further, in a case in which the determination unit 16 estimates that the occurrence cause of the artifact is the reflection of the side lobe from the reflector in the subject, the apparatus control unit 18A directs the transducer array 31 to transmit the ultrasound beam such that the side lobe is not reflected from the reflector in the subject. For example, the apparatus control unit 18A controls the transmitting and receiving circuit 32 such that beamforming conditions and the like are changed to change the tomographic plane of the subject to be imaged. The apparatus control unit 18A can perform this process on the basis of, for example, an instruction from the user through the input device 19 or can perform this process automatically. As described above, the apparatus control unit 18A directs the transducer array 31 to transmit the ultrasound beam such that the side lobe is not reflected from the reflector in the subject. Therefore, the user can easily reduce the artifacts.

### Embodiment 3

In Embodiments 1 and 2, at least one of the orientation or inclination of the ultrasound probe is decided by the user's determination in order to eliminate the artifact. However, the ultrasound diagnostic system according to the invention can automatically guide the user to determine at least one of the orientation or inclination of the ultrasound probe for eliminating the artifact.

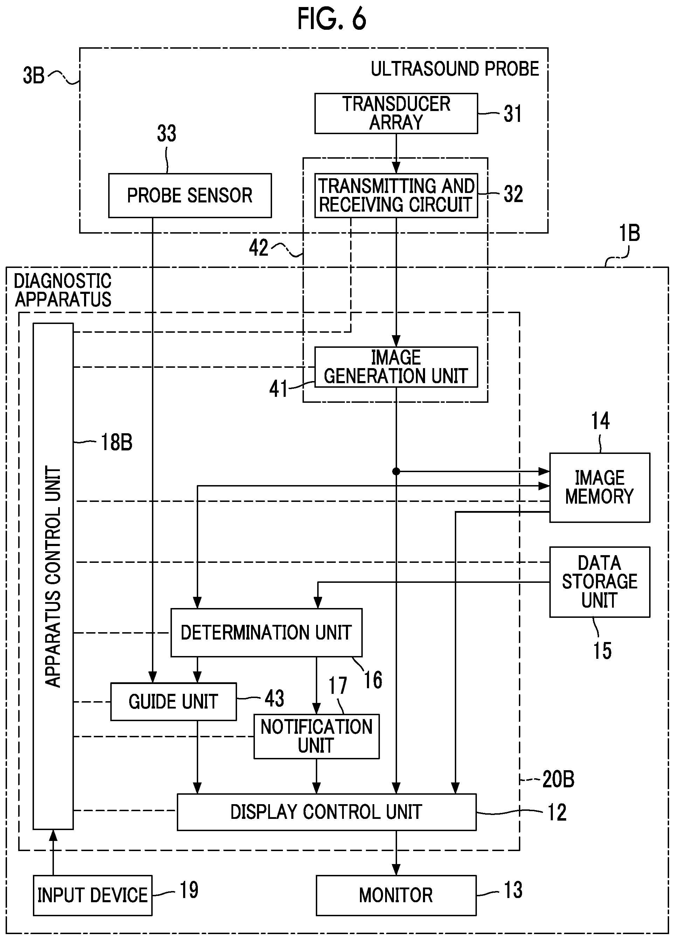

Fig. 6 illustrates a configuration of an ultrasound diagnostic system according to Embodiment 3 of the invention. The ultrasound diagnostic system according to Embodiment 3 differs from the ultrasound diagnostic system according to Embodiment 2 illustrated in Fig. 3 in that it comprises an ultrasound probe 3B instead of the ultrasound probe 3A and comprises a diagnostic apparatus 1B instead of the diagnostic apparatus 1A.

The ultrasound probe 3B differs from the ultrasound probe 3A according to Embodiment 2 in that a probe sensor 33 is added. In addition, the diagnostic apparatus 1B differs from the diagnostic apparatus 1A according to Embodiment 2 in that it comprises an apparatus control unit 18B instead of the apparatus control unit 18A and a guide unit 43 is added.

In the diagnostic apparatus 1B, the guide unit 43 is connected to the determination unit 16. The guide unit 43 is connected to the probe sensor 33 of the ultrasound probe 3B. In addition, the guide unit 43 is connected to the display control unit 12 and the apparatus control unit 18B. Further, the image generation unit 41, the display control unit 12, the determination unit 16, the notification unit 17, the apparatus control unit 18B, and the guide unit 43 of the diagnostic apparatus 1B constitute a processor 20B for the diagnostic apparatus 1B.

The probe sensor 33 is a sensor device that is disposed in the ultrasound probe 3B and detects the orientation and inclination of the ultrasound probe 3B. The probe sensor 33 can be configured to include, for example, a sensor device, such as a so-called acceleration sensor, a so-called gyro sensor, or a so-called magnetic sensor.

The guide unit 43 guides the user to change at least one of the orientation or inclination of the ultrasound probe 3B such that the artifacts are reduced, on the basis of the orientation and inclination of the ultrasound probe 3B detected by the probe sensor 33 and the occurrence location and occurrence cause of the artifact estimated by the determination unit 16. The guide unit 43 can display, for example, a message "In a case in which the orientation of the probe is rotated by 90 degrees and scanning is performed, there is a possibility that artifacts will be reduced" on the monitor 13 to guide the user to change at least one of the orientation or inclination of the ultrasound probe 3B.

As described above, according to the ultrasound diagnostic system of Embodiment 3 of the invention, the guide unit 43 guides the user to change at least one of the orientation or inclination of the ultrasound probe 3B such that the artifacts are reduced, on the basis of the orientation and inclination of the ultrasound probe 3B detected by the probe sensor 33 and the occurrence location and occurrence cause of the artifact estimated by the determination unit 16. Therefore, the user can appropriately change at least one of the orientation or inclination of the ultrasound probe 3B to easily reduce artifacts, regardless of the skill level of the user.

In addition, the configuration in which the guide unit 43 displays a message on the monitor 13 to guide the user has been described. However, a guide method is not particularly limited. For example, in a case in which the diagnostic apparatus 1B comprises a speaker (not illustrated), the guide unit 43 can output a voice through the speaker to guide the user.

### Embodiment 4

In Embodiment 3, the probe sensor 33 is provided as a probe detection unit that detects the orientation and inclination of the ultrasound probe 3B. However, the configuration of the probe detection unit is not limited thereto. The probe detection unit may be configured to, for example, capture an optical image of the ultrasound probe 3B and to analyze the captured optical image to detect the orientation and inclination of the ultrasound probe 3B.

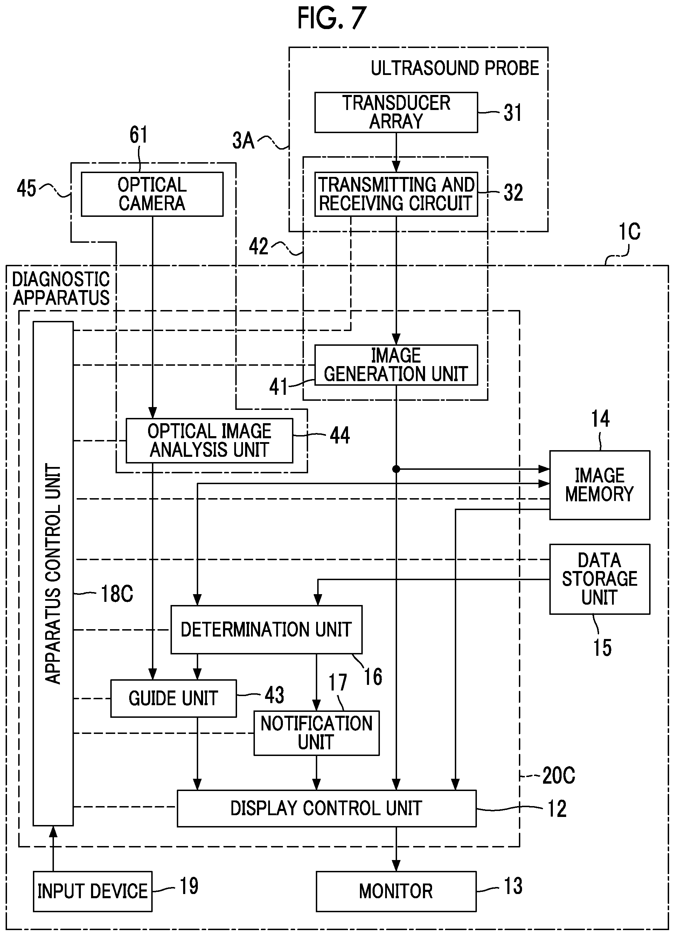

Fig. 7 illustrates a configuration of an ultrasound diagnostic system according to Embodiment 4 of the invention. The ultrasound diagnostic system according to Embodiment 4 differs from the ultrasound diagnostic system according to Embodiment 2 illustrated in Fig. 3 in that it comprises a diagnostic apparatus 1C instead of the diagnostic apparatus 1A and an optical camera 61 is added.

The diagnostic apparatus 1C differs from the diagnostic apparatus 1A according to Embodiment 2 in that it comprises an apparatus control unit 18C instead of the apparatus control unit 18A and a guide unit 43 and an optical image analysis unit 44 are added. The guide unit 43 according to Embodiment 4 is the same as the guide unit 43 according to Embodiment 3 illustrated in Fig. 6.

In the diagnostic apparatus 1C, the optical image analysis unit 44 is connected to the optical camera 61. The optical camera 61 and the optical image analysis unit 44 constitute a probe detection unit 45. In addition, the optical image analysis unit 44 is connected to the apparatus control unit 18C and the guide unit 43. Further, the image generation unit 41, the display control unit 12, the determination unit 16, the notification unit 17, the apparatus control unit 18C, the guide unit 43, and the optical image analysis unit 44 constitute a processor 20C for the diagnostic apparatus 1C.

The probe detection unit 45 which is composed of the optical camera 61 and the optical image analysis unit 44 detects the orientation and inclination of the ultrasound probe 3A.

The optical camera 61 includes an image sensor, such as a so-called charge coupled device (CCD) image sensor or a so-called complementary metal-oxide-semiconductor (CMOS) image sensor, images the ultrasound probe 3A, and acquires an optical image including the ultrasound probe 3A. The optical camera 61 transmits the acquired optical image to the optical image analysis unit 44.

The optical image analysis unit 44 analyzes the optical image acquired by the optical camera 61 to detect the orientation and inclination of the ultrasound probe. The optical image analysis unit 44 can store, for example, a plurality of template images obtained by imaging the ultrasound probe 3A at various angles, search the optical image with a so-called template matching method using these template images to detect the ultrasound probe 3A, and detect the orientation and inclination of the detected ultrasound probe 3A.

In addition, for example, the optical image analysis unit 44 can detect the orientation and inclination of the ultrasound probe 3A, using a trained model which has been trained using a plurality of template images obtained by imaging the ultrasound probe 3A at various angles as so-called training data. For example, a model that follows a ResNet algorithm, a DenseNet algorithm, an AlexNet algorithm, a Baseline algorithm, a batch normalization algorithm, a dropout regularization algorithm, a NetWidth search algorithm, a NetDepth search algorithm, or the like can be used as the trained model. Further, an appropriate combination of models that follow these algorithms can also be used as the optical image analysis unit 44.

The optical image analysis unit 44 transmits the detected orientation and inclination of the ultrasound probe 3Ato the guide unit 43.

The guide unit 43 guides the user to change at least one of the orientation or inclination of the ultrasound probe 3A such that artifacts are reduced on the basis of the orientation and inclination of the ultrasound probe 3A detected by the optical image analysis unit 44 and the occurrence location and occurrence cause of the artifact estimated by the determination unit 16.

As described above, according to the ultrasound diagnostic system of Embodiment 4 of the invention, the optical image analysis unit 44 detects the orientation and inclination of the ultrasound probe 3A on the basis of the optical image of the ultrasound probe 3A acquired by the optical camera 61, and the guide unit 43 guides the user to change at least one of the orientation or inclination of the ultrasound probe 3A such that artifacts are reduced on the basis of the orientation and inclination of the ultrasound probe 3A detected by the optical image analysis unit 44 and the occurrence location and occurrence cause of the artifact estimated by the determination unit 16. Therefore, as in the ultrasound diagnostic system according to Embodiment 3, the user can appropriately change at least one of the orientation or inclination of the ultrasound probe 3A to easily reduce artifacts, regardless of the skill level of the user.

### Embodiment 5

In Embodiment 1, the diagnostic apparatus 1 comprises the data storage unit 15. However, the data storage unit 15 can be comprised in, for example, a so-called server.

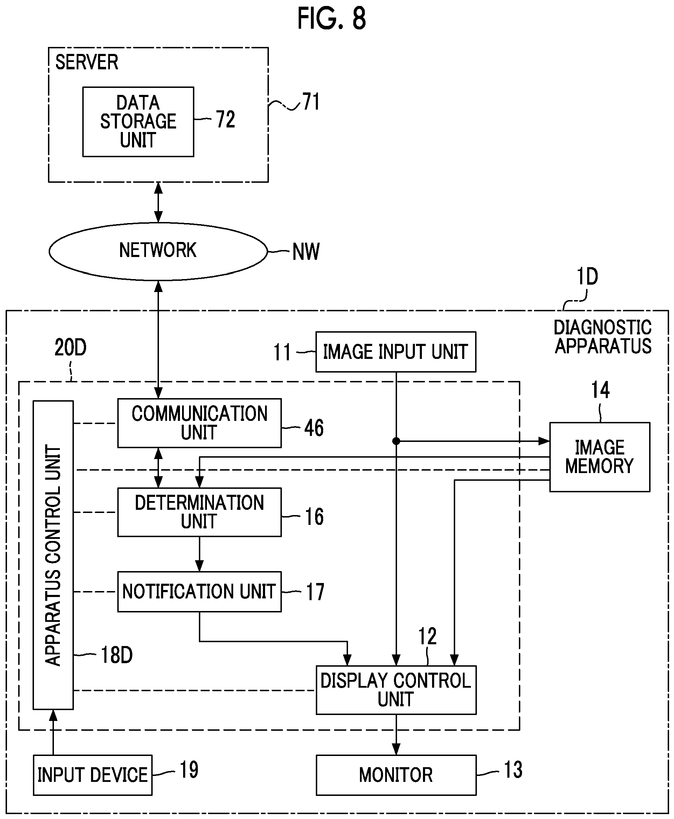

Fig. 8 illustrates a configuration of an ultrasound diagnostic system according to Embodiment 5 of the invention. The ultrasound diagnostic system according to Embodiment 5 differs from the ultrasound diagnostic system including the diagnostic apparatus 1 illustrated in Fig. 1 in that it comprises a diagnostic apparatus 1D instead of the diagnostic apparatus 1 and a server 71 connected to the diagnostic apparatus 1D through a network NW is added.

The diagnostic apparatus 1D differs from the diagnostic apparatus 1 according to Embodiment 1 in that it comprises an apparatus control unit 18D instead of the apparatus control unit 18, a communication unit 46 is added, and the data storage unit 15 is removed. The communication unit 46 is connected to the server 71 through the network NW. Further, the communication unit 46 is connected to the determination unit 16 and the apparatus control unit 18D. In addition, the display control unit 12, the determination unit 16, the notification unit 17, the apparatus control unit 18D, and the communication unit 46 constitute a processor 20D for the diagnostic apparatus 1D.

The server 71 is configured by, for example, a computer that can be connected to the network NW and is disposed at a location distant from the diagnostic apparatus 1D. The server 71 includes a data storage unit 72.

Similarly to the data storage unit 15 according to Embodiment 1, the data storage unit 72 is a memory that stores in advance the occurrence principle of each of a plurality of different types of artifacts, an artifact elimination method based on the occurrence principle, and a case in which the artifact has been effectively utilized for diagnosis. The artifact elimination method and the case in which the artifact has been effectively utilized, which are stored in the data storage unit 72, are transmitted to the communication unit 46 through the network NW. For example, a recording medium, such as a flash memory, an HDD, an SSD, an FD, a MO disk, an MT, a RAM, a CD, a DVD, an SD card, or a USB memory, is used as the data storage unit 72.

The communication unit 46 is connected to the server 71 through the network NW and transmits and receives information between the diagnostic apparatus 1D and the server 71. The communication unit 46 includes, for example, a connection terminal for wired connection to the network NW through a communication cable (not illustrated) or the like or an antenna for wireless connection to the network NW.

The determination unit 16 transmits, to the notification unit 17, the artifact elimination method and the case in which the artifact has been effectively utilized which correspond to the estimated occurrence cause of the artifact and have been transmitted from the server 71 to the communication unit 46.

The notification unit 17 notifies the user of the artifact elimination method or the case in which the artifact has been effectively utilized which has been transmitted from the determination unit 16.

As described above, even in a case in which the data storage unit 72 is included in the server 71 as in the ultrasound diagnostic system according to Embodiment 5 of the invention, the notification unit 17 notifies the user of the artifact elimination method and the case in which the artifact has been effectively utilized, similarly to the case in which the data storage unit 15 is included in the diagnostic apparatus 1 as in Embodiment 1. Therefore, the user can easily reduce the artifacts and effectively utilize the artifacts for the diagnosis of the subject.

Further, the application of the aspect of Embodiment 5 to Embodiment 1 has been described. However, the aspect of Embodiment 5 can also be applied to Embodiments 2 to 4. That is, the server 71 can be provided in the ultrasound diagnostic systems according to Embodiments 2 to 4 in the same manner as that in the ultrasound diagnostic system according to Embodiment 5.

### Embodiment 6

In Embodiment 5, the image input unit 11 and the determination unit 16 are comprised in the diagnostic apparatus 1D. However, the image input unit 11 and the determination unit 16 may be comprised in the server 71.

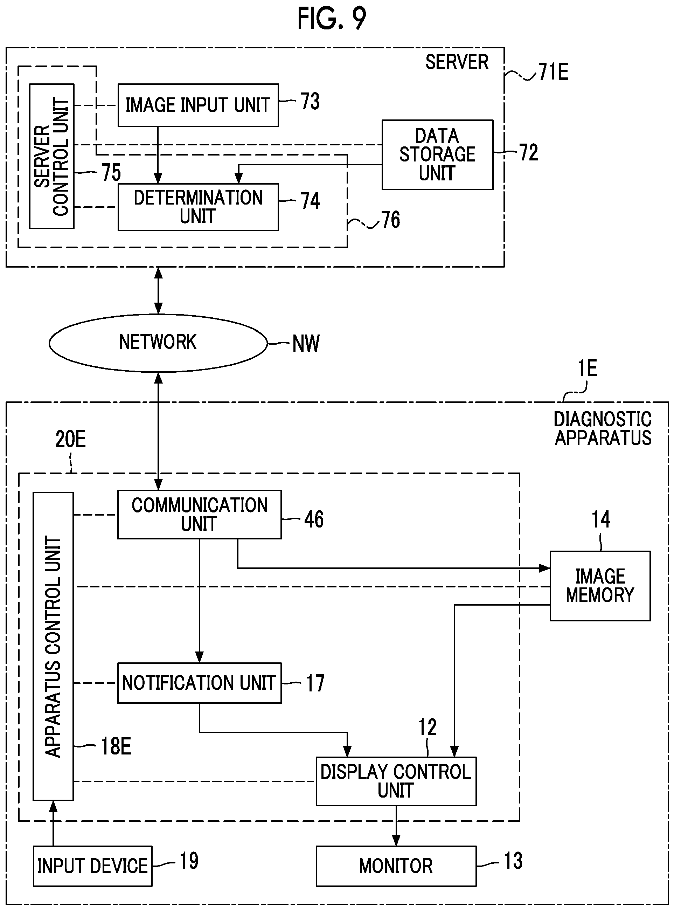

Fig. 9 illustrates a configuration of an ultrasound diagnostic system according to Embodiment 6. The ultrasound diagnostic system according to Embodiment 6 differs from the ultrasound diagnostic system according to Embodiment 5 illustrated in Fig. 8 in that it comprises a server 71E instead of the server 71 and comprises a diagnostic apparatus 1E instead of the diagnostic apparatus 1D. The diagnostic apparatus 1E and the server 71E are connected to each other through the network NW.

The diagnostic apparatus 1E differs from the diagnostic apparatus 1D according to Embodiment 5 in that it comprises an apparatus control unit 18E instead of the apparatus control unit 18D and the image input unit 11 and the determination unit 16 are removed. The display control unit 12, the notification unit 17, the apparatus control unit 18E, and the communication unit 46 constitute a processor 20E for the diagnostic apparatus 1E.

The server 71E differs from the server 71 according to Embodiment 5 in that an image input unit 73, a determination unit 74, and a server control unit 75 are added. In the server 71E, the determination unit 74 is connected to the data storage unit 72 and the image input unit 73. Further, the server control unit 75 is connected to the data storage unit 72, the image input unit 73, and the determination unit 74. In addition, the determination unit 74 and the server control unit 75 constitute a processor 76 for the server 71E.

The image input unit 73 of the server 71E is the same as the image input unit 11 of the diagnostic apparatus 1D according to Embodiment 5, and the determination unit 74 is the same as the determination unit 16 of the diagnostic apparatus 1D according to Embodiment 5.

The server control unit 75 controls each unit of the server 71E according to, for example, a program recorded in advance.

Further, the processor 76 that is composed of the determination unit 74 and the server control unit 75 of the server 71E is configured by a CPU and a control program that causes the CPU to perform various types of processes. However, the processor 76 may be configured using an FPGA, a DSP, an ASIC, a GPU, or other ICs or may be configured by combinations thereof.

In addition, the determination unit 74 and the server control unit 75 of the processor 76 may be configured to be partially or wholly integrated into, for example, one CPU.

In the ultrasound diagnostic system according to Embodiment 6, an ultrasound image is input to the image input unit 73 of the server 71E from, for example, an external apparatus (not illustrated) or a recording medium. The image input unit 73 transmits the ultrasound image input from, for example, the external apparatus or the recording medium to the determination unit 74.

The determination unit 74 determines whether or not an artifact has occurred in the ultrasound image transmitted from the image input unit 73 using the trained model. In addition, the determination unit 74 estimates the occurrence location and occurrence cause of the artifact in the ultrasound image using the trained model.

The ultrasound image input to the image input unit 73, the determination result of the determination unit 74 related to whether an artifact is present or absent, and the occurrence location and occurrence cause of the artifact estimated by the determination unit 74 are transmitted to the communication unit 46 of the diagnostic apparatus 1E through the network NW.

The communication unit 46 of the diagnostic apparatus 1E transmits the ultrasound image received from the server 71E through the network NW to the image memory 14. Then, the ultrasound image is stored in the image memory 14. Further, the ultrasound image stored in the image memory 14 is transmitted to the display control unit 12 and displayed on the monitor 13 under the control of the apparatus control unit 18E.

In addition, the communication unit 46 transmits the determination result of whether an artifact is present or absent and the occurrence location and occurrence cause of the artifact, which have been received from the server 71E through the network NW, to the notification unit 17.

In a case in which the determination unit 74 determines that an artifact has occurred, the notification unit 17 notifies the user of the occurrence location and occurrence cause of the artifact estimated by the determination unit 74. In addition, in a case in which the notification unit 17 notifies the user of the occurrence cause of the artifact, the notification unit 17 can also notify the user of an artifact elimination method based on the occurrence principle of the artifact stored in the data storage unit 72. Further, in a case in which the notification unit 17 notifies the user of the occurrence cause of the artifact, the notification unit 17 can also notify the user of the case in which the artifact has been effectively utilized for diagnosis which is stored in the data storage unit 72.

As described above, even in a case in which the image input unit 73 and the determination unit 74 are included in the server as in the ultrasound diagnostic system according to Embodiment 6 of the invention, the notification unit 17 notifies the user of the artifact elimination method or the case in which the artifact has been effectively utilized for diagnosis, similarly to a case in which the image input unit 11 and the determination unit 16 are included in the diagnostic apparatus 1D as in Embodiment 5. Therefore, the user can easily reduce artifacts and effectively utilize the artifacts for the diagnosis of the subject.

In addition, in the ultrasound diagnostic system according to Embodiment 6, the determination unit 74 is included in the server 71E. Therefore, it is necessary to perform the determination of whether an artifact is present or absent and the estimation of the occurrence location and occurrence cause of the artifact in the diagnostic apparatus 1E. As a result, it is possible to reduce the calculation load of the diagnostic apparatus 1E. Therefore, for example, in a case in which a plurality of diagnostic apparatuses 1E are installed at different locations and the user at each location diagnoses the subject using each diagnostic apparatus 1E, it is not necessary to install the expensive processor 20E with high computing capability in each diagnostic apparatus 1E. As described above, it is not necessary to provide the expensive processor 20E in the diagnostic apparatus 1E. Therefore, particularly, in a case in which a plurality of diagnostic apparatus 1E are installed, it is easy to introduce an ultrasound diagnostic system.

### Explanation of References

1, 1A, 1B, 1C, 1D, 1E: diagnostic apparatus
3A, 3B: ultrasound probe
11, 11A, 73: image input unit
12: display control unit
13: monitor
14: image memory
15, 72: data storage unit
16, 74: determination unit
17: notification unit
18, 18A, 18B, 18C, 18D, 18E: apparatus control unit
19: input device
20, 20A, 20B, 20C, 20D, 20E, 76: processor
31: transducer array
32: transmitting and receiving circuit
33: probe sensor
41: image generation unit
42: image acquisition unit
43: guide unit
44: optical image analysis unit
45: probe detection unit
46: communication unit
51: pulser
52: amplification unit
53: AD conversion unit
54: beam former
55: signal processing unit
56: DSC
57: image processing unit
61: optical camera
71, 71E: server
75: server control unit
NW: network

## Claims

1. An ultrasound diagnostic system comprising:
an image input unit (11, 11A, 73) that inputs an ultrasound image;
a determination unit (16, 74) that determines whether or not an artifact has occurred in the ultrasound image input to the image input unit (11, 11A, 73), using a trained model trained using learning data in which a plurality of ultrasound images including artifacts and occurrence causes of the artifacts are associated with each other; and
a notification unit (17) that gives a notification of an occurrence location and an occurrence cause of the artifact in the ultrasound image in a case in which the determination unit (16, 74) determines that the artifact has occurred.

2. The ultrasound diagnostic system according to claim 1, further comprising:
a data storage unit (15, 72) that stores an occurrence principle of the artifact and an artifact elimination method based on the occurrence principle,
wherein, in a case in which the notification unit (17) gives the notification of the occurrence cause, the notification unit (17) also gives a notification of the artifact elimination method based on the occurrence principle which is stored in the data storage unit (15, 72).

3. The ultrasound diagnostic system according to claim 2,
wherein the data storage unit (15, 72) stores a case in which an artifact has been effectively utilized for diagnosis, and
in a case in which the notification unit (17) gives the notification of the occurrence cause, the notification unit (17) also gives a notification of the case in which the artifact has been effectively utilized for diagnosis which is stored in the data storage unit (15, 72).

4. The ultrasound diagnostic system according to claim 2 or 3, further comprising:
a diagnostic apparatus (1, 1A, 1B, 1C) that includes the image input unit (11, 11A), the determination unit (16), and the notification unit (17),
wherein the data storage unit (15) is disposed in the diagnostic apparatus (1, 1A, 1B, 1C).

5. The ultrasound diagnostic system according to claim 2 or 3, further comprising:
a diagnostic apparatus (1D) that includes the image input unit (11, 11A), the determination unit (16), and the notification unit (17); and
a server (71) that is connected to the diagnostic apparatus through a network (NW),
wherein the data storage unit (72) is disposed in the server (71).

6. The ultrasound diagnostic system according to claim 2 or 3, further comprising:
a diagnostic apparatus (1E) that includes the notification unit (17); and
a server (71E) that is connected to the diagnostic apparatus (1E) through a network (NW) and includes the image input unit (73) and the determination unit (74),
wherein the data storage unit (72) is disposed in the server (71E).

7. The ultrasound diagnostic system according to any one of claims 1 to 6, further comprising:
an input device (19) that is used to designate a region of interest in response to an input operation of a user on the ultrasound image input to the image input unit (11, 11A, 73),
wherein the determination unit (16, 74) determines whether or not an artifact has occurred in the region of interest.

8. The ultrasound diagnostic system according to any one of claims 1 to 7,
wherein the image input unit (11A) includes
an ultrasound probe (3A, 3B) having a transducer array (31) and
an image acquisition unit (42) that transmits and receives an ultrasound beam to and from a subject with the transducer array (31) and acquires an ultrasound image on the basis of a received signal output from the transducer array (31).

9. The ultrasound diagnostic system according to claim 8, further comprising:
a probe detection unit (33, 45) that detects an orientation and an inclination of the ultrasound probe (3A, 3B); and
a guide unit (43) that guides changing at least one of the orientation or the inclination of the ultrasound probe (3A, 3B) such that the artifacts are reduced on the basis of the orientation and the inclination of the ultrasound probe (3A, 3B) detected by the probe detection unit (33, 45).

10. The ultrasound diagnostic system according to claim 9,
wherein the probe detection unit (33) consists of a probe sensor (33) that is disposed in the ultrasound probe (3B).

11. The ultrasound diagnostic system according to claim 9,
wherein the probe detection unit (45) includes
an optical camera (61) that images the ultrasound probe (3A) to acquire an optical image and
an optical image analysis unit (44) that analyzes the optical image acquired by the optical camera (61) to detect the orientation and the inclination of the ultrasound probe (3A).

12. A method for controlling an ultrasound diagnostic system, the method comprising:
inputting an ultrasound image;
determining whether or not an artifact has occurred in the input ultrasound image, using a trained model trained using learning data in which a plurality of ultrasound images including artifacts and occurrence causes of the artifacts are associated with each other; and
giving a notification of an occurrence location and an occurrence cause of the artifact in the ultrasound image in a case in which it is determined that the artifact has occurred.
